# EUROPÄISCHE PATENTSCHRIFT

(11) **EP 3 340 858 B1**
(45) Veröffentlichungstag und Bekanntmachung des Hinweises auf die Patenterteilung: **10.01.2024**
(21) Anmeldenummer: 16760413.1
(22) Anmeldetag: 23.08.2016
(51) Int. Cl.: A61B 5/00, A61B 5/11

(54) **ERFASSEN UND AUSWERTEN VON BEWEGUNGEN EINES BENUTZERS**
ACQUISITION AND EVALUATION OF MOVEMENTS OF A USER
ACQUISITION ET EVALUATION DES MOVEMENTS D'UN USAGER

(30) Priorität: 24.08.2015 CH 12162015
(43) Veröffentlichungstag der Anmeldung: 04.07.2018
(73) Patentinhaber: Tyromotion GmbH, 8020 Graz (AT); ETH Zürich, 8092 Zürich (CH); Universität Zürich, 8006 Zürich (CH); Zürcher Hochschule Der Künste, 8031 Zürich (CH)
(72) Erfinder: FRANINOVIC, Karmen, 8049 Zürich (CH); MEIER, Dinis, 8003 Zürich (CH); BAUER, Samuel, 8004 Zürich (CH); GASSERT, Roger, 8620 Wetzikon (CH); KIM, Yeongmi, 6020 Innsbruck (AT); LEUENBERGER, Kaspar, 8048 Zürich (CH); LUFT, Andreas, 8142 Uitikon Waldegg (CH); HELD, Jeremia, 8053 Zürich (CH)
(74) Vertreter: Maikowski & Ninnemann Patentanwälte Partnerschaft mbB
(86) Internationale Anmeldenummer: PCT/EP2016/069870
(87) Internationale Veröffentlichungsnummer: WO 2017/032765

(56) Entgegenhaltungen:
- EP-A1- 1 366 712
- WO-A1-2014/113143
- WO-A1-2014/207294
- US-A1- 2006 052 727
- US-A1- 2014 378 872
- US-A1- 2015 190 084
- US-A1- 2015 198 460

## Beschreibung

### Technisches Gebiet

Die Erfindung betrifft eine Vorrichtung gemäss Anspruch 1 sowie ein Verfahren gemäss Anspruch 11.

Solche Vorrichtungen, die eine Recheneinheit, einen Datenspeicher, eine Sensoreinheit mit mindestens einem biometrischen Sensor und eine Befestigungseinrichtung zur Befestigung der Sensoreinheit an einem Arm des Benutzers umfassen, wobei die Recheneinheit zur Erfassung von Bewegungsdaten aus durch die Sensoreinheit erzeugten Bewegungssignalen und zur Speicherung der Bewegungsdaten im Datenspeicher ausgestaltet ist, können zur Erfassung und Auswertung von Bewegungen eines Benutzers eingesetzt werden.

### Stand der Technik

Zur Therapie oder zum Training von Personen ist es in vielen Anwendungen von Bedeutung die Bewegungen der Personen zu erfassen und auszuwerten. Beispielsweise sind in den vergangen Jahren diverse Geräte zum Messen der Aktivität von Sportlern beziehungsweise Fitnessinteressierten auf den Markt gekommen. Dabei messen und dokumentieren diese Geräte verschiedene Parameter wie beispielsweise Anzahl Schritte, zurückgelegte Distanz etwa aufgrund von ermittelten (Global Positioning System (GPS) Daten oder den Puls. Beispielsweise sind in der US 2015/0198460 A1 und der EP 1 366 712 A1 solche Geräte beschrieben. Mit fortschreitender Miniaturisierung von Sensoren werden künftig wohl weitere dazukommen.

Häufig besteht aber ein Bedürfnis nach spezifischeren Bewegungen der Person in der Therapie oder beim Training. Beispielsweise ist es bei der Therapie von Schlaganfallpatienten von zentraler Bedeutung, dass die betroffene Person bestimmte Körperteile trainiert. Insbesondere ist die häufigste Folge eines Schlaganfalls, dass die betroffene Person eine halbseitige Lähmung des Körpers erfährt. Die Rehabilitation kann mehrere Jahre dauern und benötigt viel Disziplin und Ausdauer. Um Fortschritt zu erzielen müssen die betroffenen Körperteile täglich unter grösster Anstrengung bewegt werden. Besonders werden häufig beeinträchtigte Arme im Alltag vernachlässigt.

Für die Erfassung und Auswertung von Bewegungen spezifischer Körperteile insbesondere von Armen und Fingern eignen sich die oben erwähnten Geräte jedoch nicht.

Der vorliegenden Erfindung liegt daher die Aufgabe zu Grunde, eine Vorrichtung oder ein Verfahren vorzuschlagen, mit dem auf effiziente Weise die Bewegungen eines Arms eines Benutzer erfasst und ausgewertet werden können.

### Darstellung der Erfindung

Die Aufgabe wird erfindungsgemäss durch eine Vorrichtung gelöst, wie sie im unabhängigen Anspruch 1 definiert ist, sowie durch ein Verfahren, wie es im unabhängigen Anspruch 11 definiert ist. Vorteilhafte Ausführungsvarianten der Erfindung ergeben sich aus den abhängigen Ansprüchen.

Das Wesen der Erfindung besteht im Folgenden: Eine Vorrichtung zur Erfassung und Auswertung von Bewegungen eines Benutzers umfasst eine Recheneinheit, einen Datenspeicher, eine Sensoreinheit mit mindestens einem biometrischen Sensor und eine Befestigungseinrichtung zur Befestigung der Sensoreinheit an einem Arm des Benutzers. Die Recheneinheit ist zur Erfassung von Bewegungsdaten aus durch die Sensoreinheit erzeugten Bewegungssignalen und zur Speicherung der Bewegungsdaten im Datenspeicher ausgestaltet. Die Vorrichtung umfasst weiter ein Auswertungsmodul, mit dem Sekundärbewegungsdaten, die passive Armbewegungen des Benutzers repräsentieren, aus den Bewegungsdaten filterbar sind.

Der Begriff "Arm" kann sich im Zusammenhang mit der Erfindung auf Arme eines Menschen in einem weiteren Sinne beziehen. Dabei kann er insbesondere sowohl Oberarm, Ellenbogen und Unterarm umfassen wie auch Handgelenk, Hand und Finger.

Der Begriff "passive Armbewegung" kann sich auf eine Bewegung des Arms des Benutzers beziehen, die durch eine unspezifische Bewegung des Körpers des Benutzers oder als Begleitbewegung erzeugt wird. Beispielsweise kann eine passive Armbewegung eine Bewegung des Arms sein, die durch eine Bewegung des gesamten Körpers des Benutzers induziert wird, wie sie etwa von einem Transportmittel auf den ganzen Körper des Benutzers inklusive seines Arms ausgeübt wird. Oder eine passive Armbewegung kann auch aus der einer Gesamtbewegung des Körpers oder der Bewegung eines anderen Körperteiles des Körpers des Benutzers resultieren. Insbesondere kann eine passive Armbewegung zum Beispiel durch ein eine Gehbewegung des Benutzers erzeugt werden.

Im Gegensatz dazu kann sich der Begriff "aktive Armbewegung" auf eine gezielte Bewegung des Arms des Benutzers beziehen. Beispielsweise könne gezielten Armbewegungen solche sein, die zum Hantieren mit einem Gegenstand oder dergleichen dienen.

Die Recheneinheit kann einen Prozessor (CPU) und einen Arbeitsspeicher (Random Access Memory (RAM)) umfassen. Sie kann zur Erfassung und Speicherung von Bewegungsdaten ausgestaltet sein, indem sie ein Computerprogramm ausführt, das die entsprechenden Schritte beziehungsweise Funktionen durchführt. Sie kann also entsprechend programmiert sein. Auch kann die Recheneinheit zu diesem Zweck geschaltet sein beziehungsweise als Schaltung ausgebildet sein, die diese Schritte und Funktionen erfüllt.

Das Auswertungsmodul kann von der Recheneinheit umfasst beziehungsweise durch diese implementiert sein. Insbesondere kann die Recheneinheit so programmiert sein, dass sie das Auswertungsmodul implementiert.

Das Auswertungsmodul ermöglicht, dass aus den von der Vorrichtung erfassten Bewegungssignalen, diejenigen identifizierbar sind, die einer aktiven Armbewegung zuzuordnen sind. Insbesondere können alle Bewegungsdaten aus dem mittels der Sensoreinheit erfassten Bewegungssignalen entfernt werden, die von einer passiven Armbewegung ausgelöst sind. Entsprechend ist die Vorrichtung fähig, aus den Bewegungsdaten die Arm- und/oder Handbewegung herauszurechnen und diese dem Benutzer zur Verfügung zu stellen. Dies kann für den Benutzer von beträchtlichem Nutzen sein, beispielsweise wenn er durch Krankheit oder Unfall in der Arm- und/oder Handfunktion beeinträchtigt ist. Auch kann dies dem Benutzer helfen, zu verstehen, wie er eine Hand oder seinen Arm über einen gewissen Zeitraum verwendet hat oder ob er inaktiv war. Insbesondere ist auch ein Vergleich des Gebrauchs des linken und des rechten Armes möglich. So ermöglicht die Vorrichtung auf effiziente Weise die Bewegungen eines Arms des Benutzers zu erfassen und auszuwerten.

Vorzugsweise umfasst die Vorrichtung eine Warneinrichtung, wobei die Recheneinheit dazu ausgestaltet ist, die gefilterten Bewegungsdaten auszuwerten und die Warneinrichtung zu aktivieren, wenn die ausgewerteten Bewegungsdaten über einen vordefinierten Ruhezeitraum einen vordefinierten Grenzwert nicht überschreiten. Auf diese Weise kann der Benutzer über eine Inaktivität informiert werden, was die Trainingseffizienz erhöhen kann.

Dabei umfasst die Warneinrichtung vorzugsweise eine grafische Anzeige, einen Lautsprecher, eine Vibrationsstruktur, einen Muskelstimulator oder eine Kombination davon. Mit solch einer Warneinrichtung kann der Benutzer effizient benachrichtigt werden. Die Vorrichtung umfasst vorzugsweise eine Montageeinrichtung zur Befestigung der Warneinrichtung an einem Finger oder einem Handballen des Benutzers. Eine solche Montageeinrichtung ermöglicht ein bequemes sicheres An- und Ausziehen durch den Benutzer.

Vorzugsweise umfasst das Auswertungsmodul einen Hochpassfilter und/oder einen Tiefpassfilter, mit dem beziehungsweise mit denen die Bewegungsdaten vorfilterbar sind. Ein solcher Hochpassfilter beziehungsweise Tiefpassfilter ermöglicht es, störende Signale kleineren Ausmasses oder Ausreisser nach oben und unten zu entfernen. Dadurch kann die Effizienz der Vorrichtung weiter gesteigert werden. Dabei ist der Hochpassfilter beziehungsweise der Tiefpassfilter des Auswertungsmoduls vorzugsweise auf einen Wert zwischen 0.1 Hz und 1 Hz und insbesondere auf einen Wert von etwa 0.3 Hz eingestellt ist. Mit einem so eingestellten Hochpassfilter beziehungsweise Tiefpassfilter, können viele Bewegungsdaten ausgefiltert werden, die für die Auswertung nicht von oder von untergeordneter Bedeutung sind.

Das Auswertungsmodul ist dazu ausgestaltet, fortlaufend Magnituden der Bewegungsdaten zu berechnen. Die Magnituden können sich auf eine Kurve der Bewegungsdaten beziehen. Dabei umfassen die Bewegungsdaten vorzugweise pro Zeitpunkt jeweils Werte von Bewegungen in drei Richtungen eines dreidimensionalen Koordinatensystems, und die Magnituden entsprechend vorzugsweise jeweils der Wurzel aus der Summe der Quadrate der Werte in den drei Richtungen des gleichen Zeitpunkts.

Das Auswertungsmodul ist dazu ausgestaltet, die aus den Bewegungsdaten berechneten Magnituden in einer Fensterfunktion zu bearbeiten. Der Begriff "Fensterfunktion" bezieht sich in diesem Zusammenhang auf eine Funktion zur Frequenzanalyse, die festlegt, mit welcher Gewichtung bei einer Abtastung eines Signals gewonnene Abtastwerte innerhalb eines Ausschnittes beziehungsweise Fensters in nachfolgende Berechnungen eingehen. Solche Fensterfunktionen sind allgemein bekannt. Dabei kann die Fensterfunktion vorteilhafterweise eine Hamming-FensterFunktion sein.

Das Auswertungsmodul ist dazu ausgestaltet, mittels Fourier-Transformation (FT) und insbesondere schneller Fourier-Transformation (FFT) periodische Muster in den mit der Fensterfunktion bearbeiteten Magnituden zu identifizieren. Unter dem Begriff "schnelle Fourier-Transformation" beziehungsweise FFT wird hier ein bekannter Algorithmus zur Berechnung der diskreten Fourier-Transformation (DFT) verstanden, mit dem ein digitales Signal in seine Frequenzanteile zerlegt und diese dann analysiert werden können. Unter Verwendung einer schnellen Fourier-Transformation können effizient periodische Muster in den Frequenzdaten beziehungsweise den Magnituden ermittelt werden.

Dabei ist das Auswertungsmodul dazu ausgestaltet ist, Schritte zu zählen, falls ein periodisches Muster identifiziert wird. Auch ist das Auswertungsmodul dazu ausgestaltet, Armaktivitäten zu zählen, falls kein periodisches Muster identifiziert wird. Auf diese Weise können die Bewegungsdaten effizient in durch Gehen entstandene Bewegungen und andere Armbewegungen triagiert beziehungsweise getrennt werden. In einer bestimmten Periode beziehungsweise in einem bestimmten Fenster der Bewegungsdaten können also entweder Schritte oder Armbewegungen gezählt werden, aber nicht beides. Dies kann eine effiziente zielführende Auswertung ermöglichen.

Das Auswertungsmodul beziehungsweise die Recheneinheit ist dabei vorzugsweise dazu ausgestaltet, einen Aktivitätszähler zu berechnen, der der Summe aller Magnituden oder Integrale in einem vordefinierten Zeitfenster entspricht. Der Aktivitätszähler kann in einer Referenzeinheit definiert sein. Dabei kann eine Bewegung, die einem Griff zu einer Türfalle etwa einer Einheit entspricht. Der Aktivitätszähler kann eine einfache Auswertung der Armbewegungen des Benutzers ermöglichen. Insbesondere kann damit berücksichtigt werden, dass eine Vielzahl von verhältnismässig kleinen Bewegungen gleich bewertet werden wie eine geringere Anzahl von verhältnismässig grossen Bewegungen.

Dabei liegt das vordefinierte Zeitfenster vorzugsweise in einem Bereich von zwischen 0.5 Sekunden bis zehn Minuten und insbesondere in einem Bereich von zwischen zwei Sekunden bis fünf Minuten. Dies ermöglicht ein effizientes zweckmässiges Aufrechnen der Bewegungsdaten und Bestimmen der Aktivitätszähler.

Vorzugsweise umfasst der mindestens eine biometrische Sensor der Sensoreinheit einen Beschleunigungssensor, ein Gyroskop, einen Magnetometer, einen Drucksensor, einen GPS-Sensor, einen Muskelaktivitätssensor wie einen EMG Muskelsensor oder eine Kombination davon. Der Begriff "EMG Muskelsensor" kann sich in diesem Zusammenhang auf einen Sensor der Elektromyografie (EMG) beziehen, mit dem die elektrische Muskelaktivität erfasst wird. Mit solchen biometrischen Sensoren kann eine aktive Armbewegung effizient erfasst und ausgewertet werden.

Vorzugsweise ist die Befestigungseinrichtung zur Befestigung der Sensoreinheit an einem Finger des Benutzers ausgestaltet. So können Bewegungen von Finger ausgewertet werden.

Bevorzugt umfasst die Vorrichtung einen Schnittstellenadapter zur Verbindung mit einem Computer. Dabei kann der Computer ein Desktop-Computer, ein Laptop-Computer, ein Tablet-Computer, ein Smartphone oder etwas ähnliches sein. Der Schnittstellenadapter ist vorzugsweise ein Funkschnittstellenadapter zur kabellosen Verbindung mit dem Computer. Mit einer solchen Vorrichtung können Daten effizient an den Computer übersendet werden. Auf dem Computer können diese weiter ausgewertet oder dargestellt oder gespeichert werden.

Vorzugsweise ist die Sensoreinheit zum Abtasten in einer Frequenz von mindestens 10 Hz oder von mindestens 20 Hz und insbesondere von in einem Bereich zwischen etwa 50 Hz bis etwa 100 Hz eingestellt. Ein Abtasten in einer solchen Frequenz ermöglicht ein effizientes Erfassen und Auswerten von Armbewegungen.

Ein anderer Aspekt der Erfindung betrifft ein Verfahren zur Erfassung und Auswertung von Bewegungen eines Benutzers. Das Verfahren umfasst die Schritte: Befestigen einer Sensoreinheit an einem Arm des Benutzers, wobei die Sensoreinheit mindestens einen biometrischen Sensor aufweist; Erfassen von Bewegungsdaten aus durch die Sensoreinheit erzeugten Bewegungssignalen; Speichern der Bewegungsdaten in einem Datenspeicher; und Ausfiltern von Sekundärbewegungsdaten, die passive Armbewegungen des Benutzers repräsentieren, aus den Bewegungsdaten umfasst. Die gefilterten Bewegungsdaten können insbesondere Primärbewegungsdaten sein. Mit einem solchen Verfahren und seinen nachstehend beschriebenen bevorzugten Ausführungsformen können die oben im Zusammenhang mit der Vorrichtung beschriebenen Effekte und Vorteile effizient realisiert werden. Dabei kann das Verfahren durch Computerprogramm implementiert sein, das Befehle zu dessen Ausführung umfasst, wenn das Computerprogramm auf der Vorrichtung läuft.

Das Verfahren umfasst vorzugsweise ein Auswerten der gefilterten Bewegungsdaten und ein Warnen des Benutzers, wenn die ausgewerteten Bewegungsdaten über einen vordefinierten Ruhezeitraum einen vordefinierten Grenzwert nicht überschreiten. Dabei kann das Warnen ein Ausgeben einer visuellen Information, eines akustischen Alarms, einer Vibration oder eine Kombination davon umfassen.

Das Verfahren umfasst vorzugsweise ein vor dem Ausfiltern der Sekundärbewegungsdaten ausgeführtes Hochpassfiltern. Dabei erfolgt das Hochpassfiltern vorzugsweise mit 0.3 Hz.

Beim Ausfiltern der Sekundärbewegungsdaten werden fortlaufend Magnituden der Bewegungsdaten berechnet. Dabei entspricht beim Berechnen der Magnituden der Bewegungsdaten pro Zeitpunkt bevorzugt jeweils die Magnitude der Wurzel aus der Summe der Quadrate von Werten in drei Richtungen eines dreidimensionalen Koordinatensystems. Das Verfahren umfasst in bevorzugter Weise ein Berechnen eines Aktivitätszählers, der der Summe aller Magnituden oder Integrale in einem vordefinierten Zeitfenster entspricht. Dabei liegt das vordefinierte Zeitfenster vorzugsweise in einem Bereich von zwischen 0.5 Sekunden bis zehn Minuten und insbesondere in einem Bereich von zwischen zwei Sekunden bis fünf Minuten.

Bevorzugt umfasst der mindestens eine biometrische Sensor der Sensoreinheit einen Beschleunigungssensor, einen Gyroskop, einen Magnetometer, einen Drucksensor oder eine Kombination davon. Vorzugsweise ist beim Verfahren die Sensoreinheit an einem Finger des Benutzers befestigt. Bevorzugt umfasst das Verfahren ein Übertragen der ausgefilterten Bewegungsdaten an einen Computer umfasst.

Vorzugsweise ist beim Verfahren die Sensoreinheit zum Abtasten in einer Frequenz von mindestens 10 Hz oder von mindestens 20 Hz und insbesondere von in einem Bereich zwischen etwa 50 Hz bis etwa 100 Hz eingestellt.

Bevorzugt umfasst das Verfahren weiter die Schritte: Befestigen einer weiteren Sensoreinheit an einem zweiten Arm des Benutzers, wobei die weitere Sensoreinheit mindestens einen biometrischen Sensor aufweist; Erfassen von weiteren Bewegungsdaten aus durch die weitere Sensoreinheit erzeugten Bewegungssignalen; Speichern der weiteren Bewegungsdaten in einem Datenspeicher; Ausfiltern von Sekundärbewegungsdaten aus den weiteren Bewegungsdaten; und Vergleichen der ausgefilterten Bewegungsdaten mit den ausgefilterten weiteren Bewegungsdaten. Auf diese Weise können Armebewegungen der beiden Arme miteinander verglichen werden. Dies ermöglicht es weiter Rückschlüsse über den Fortschritt der Armbewegungen zu ziehen und dem Benutzer näher zu bringen.

Vorzugsweise umfasst das Verfahren ein Definieren eines Bewegungsziels, insbesondere eines Tages- oder Wochenbewegungsziels und ein Darstellen eines Grads der Erreichung des Bewegungsziels. Auf diese Weise kann der Benutzer effizient dazu motiviert werden, das Bewegungsziel einzuhalten, womit ein verbesserter Fortschritt ermöglicht wird. Dabei kann das Bewegungsziel aufgrund von gespeicherten Bewegungsdaten automatisch definiert und insbesondere automatisch nach oben angepasst werden.

### Kurze Beschreibung der Zeichnungen

Weitere vorteilhafte Ausgestaltungen der Erfindung ergeben sich aus der nachfolgenden Beschreibung von Ausführungsbeispielen der Erfindung mit Hilfe der schematischen Zeichnung. Insbesondere werden im Folgenden die erfindungsgemässe Vorrichtung und das erfindungsgemässe Verfahren unter Bezugnahme auf die beigefügten Zeichnungen anhand von Ausführungsbeispielen detaillierter beschrieben. Es zeigen:
- Fig. 1: eine perspektivische Ansicht eines Ausführungsbeispiels einer erfindungsgemässen Vorrichtung und einem mit ihr verbundenem Smartphone;
- Fig. 2: eine perspektivische Ansicht der Vorrichtung von Fig. 1 in einem getrennten Zustand; und
- Fig. 3: eine Kurve von mittels der Vorrichtung von Fig. 1 erfassten Bewegungsdaten.

### Weq(e) zur Ausführung der Erfindung

Bestimmte Ausdrücke werden in der folgenden Beschreibung aus praktischen Gründen verwendet und sind nicht einschränkend zu verstehen. Die Wörter "rechts", "links", "unten" und "oben" bezeichnen Richtungen in der Zeichnung, auf die Bezug genommen wird. Die Ausdrücke "nach innen", "nach aussen" "unterhalb", "oberhalb", "links", "rechts" oder ähnliche werden zur Beschreibung der Anordnung bezeichneter Teile zueinander, der Bewegung bezeichneter Teile zueinander und der Richtungen hin zum oder weg vom geometrischen Mittelpunkt der Vorrichtung sowie benannter Teile derselben wie in den Fig. dargestellt verwendet. Diese räumlichen Relativangaben umfassen auch andere Positionen und Ausrichtungen als die in den Fig. dargestellten. Zum Beispiel wenn ein in den Fig. dargestelltes Teil umgedreht wird, sind Elemente oder Merkmale, die als "unterhalb" beschrieben sind, dann "oberhalb". Die Terminologie umfasst die oben ausdrücklich erwähnten Wörter, Ableitungen von denselben und Wörter ähnlicher Bedeutung.

Um Wiederholungen in den Fig. und der zugehörigen Beschreibung der verschiedenen Aspekte und Ausführungsbeispielen zu vermeiden, sollen bestimmte Merkmale als gemeinsam für verschieden Aspekte und Ausführungsbeispiele verstanden werden. Das Weglassen eines Aspekts in der Beschreibung oder einer Fig. lässt nicht darauf schliessen, dass dieser Aspekt in dem zugehörigen Ausführungsbeispiel fehlt. Vielmehr kann ein solches Weglassen der Klarheit und dem Verhindern von Wiederholungen dienen. In diesem Zusammenhang gilt für die gesamte weitere Beschreibung folgende Festlegung: Sind in einer Figur zum Zweck zeichnerischer Eindeutigkeit Bezugszeichen enthalten, aber im unmittelbar zugehörigen Beschreibungstext nicht erwähnt, so wird auf deren Erläuterung in vorangehenden Figurenbeschreibungen Bezug genommen. Sind ausserdem im unmittelbar zu einer Figur gehörigen Beschreibungstext Bezugszeichen erwähnt, die in der zugehörigen Figur nicht enthalten sind, so wird auf die vorangehenden und nachstehenden Figuren verwiesen. Ähnliche Bezugszeichen in zwei oder mehreren Fig. stehen für ähnliche oder gleiche Elemente.

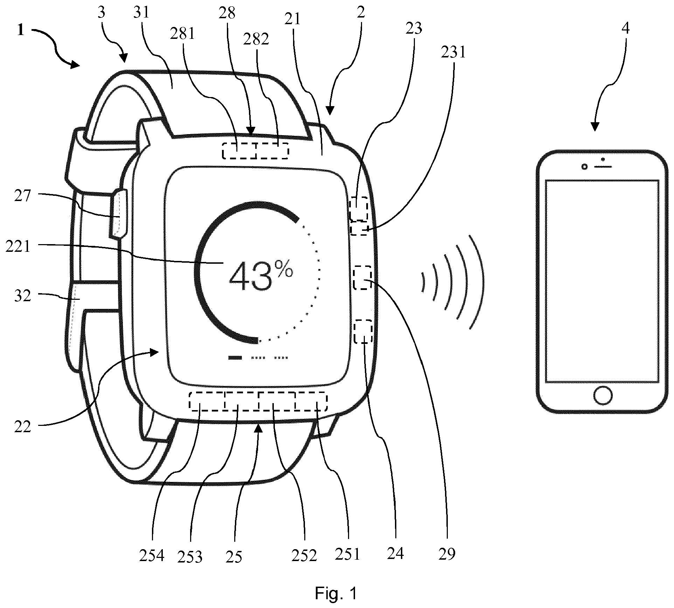

Fig. 1 zeigt eine erfindungsgemässe Vorrichtung 1 mit einem Funktionsteil 2 und einem Armband 3 als Befestigungseinrichtung und als Montageeinrichtung. Das Funktionsteil 2 umfasst ein Gehäuse 21, in das ein Display 22 als visuelle Anzeige eingelassen ist. Im Innern des Gehäuses 21 sind eine Recheneinheit 23, ein Datenspeicher 24, eine Sensoreinheit 25, eine Warneinrichtung 28 und ein Funkschnittstellenadapter 29 angeordnet. Auf der linken Seite ist das Gehäuse 21 mit einem Druckknopf 27 ausgestattet, über den das Funktionsteil von einem Benutzer bedient werden kann.

Die Sensoreinheit 25 umfasst mehrere biometrische Sensoren, nämlich einen GPS-Sensor 251, ein Gyroskop 252, einen Beschleunigungssensor 253 und ein Magnetometer 254. Die Warneinrichtung 28 umfasst einen Lautsprecher 282, einen Vibrator 281 und das Display 22. Die Recheneinheit 23 ist dazu programmiert, ein Auswertungsmodul 231 zu implementieren.

Das Armband 3 umfasst ein Kunststoffband 31 und einen Verschluss 32. Über den Verschluss 32 kann das Kunststoffband 31 in seiner Länge eingestellt werden, so dass die Vorrichtung 1 mit seiner Sensoreinheit 25 an einem Handgelenk des Benutzers befestigt werden kann.

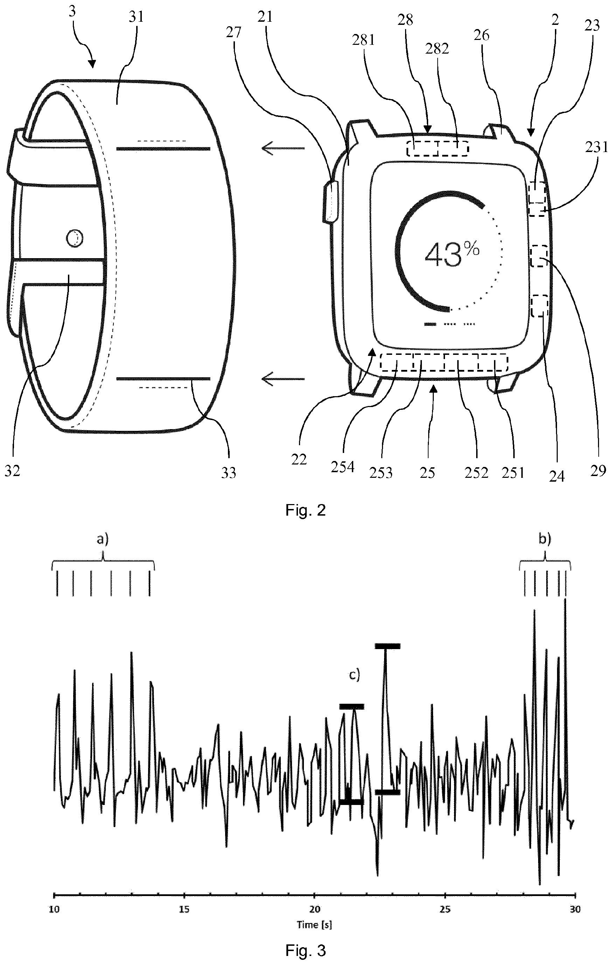

Wie in Fig. 2 gezeigt kann die Funktionseinheit 2 vom Armband 3 getrennt und wieder an ihm montiert werden. Dazu weist das Gehäuse 21 eine Klippstruktur 26 auf und das Kunststoffband 31 eine Klippführung 33. Die Klippstruktur 26 umfasst zwei obere und zwei untere Klemmteile, wobei das Kunststoffband 21 jeweils seitlich zwischen zwei der Klemmteile einklemmbar ist.

Im Betrieb wird die Vorrichtung 1 am Handgelenk des Benutzers befestigt und die biometrischen Sensoren der Sensoreinheit 25 erfassen in einer Frequenz von zwischen 50 Hz und 100 Hz verschiedene Bewegungsparameter des Arms des Benutzers. Daraus erzeugen sie Bewegungssignale, die an die Recheneinheit 23 weitergeleitet werden. Die Recheneinheit 23 erzeugt aus den Bewegungssignalen Daten und speichert diese im Datenspeicher 24.

Zudem aggregiert die Recheneinheit 23 die Bewegungssignale der biometrischen Sensoren der Sensoreinheit 25 zu Bewegungsdaten, die sie ebenfalls im Datenspeicher 24 speichert. Dabei filtert das Auswertungsmodul 231 der Recheneinheit 23 mit einem auf 0.3 Hz eingestellten Hochpassfilter Ausreisser in den Bewegungsdaten aus. Danach filtert es Sekundärbewegungsdaten, die passive Armbewegungen des Benutzers repräsentieren, aus den Bewegungsdaten.

In Fig. 3 ist ein Verlauf von ungefilterten Bewegungsdaten gezeigt. Dabei ist ersichtlich, dass bis etwa 14 Sekunden regelmässige Peaks a) auftreten, wie sie typischerweise beim Gehen oder Laufen des Benutzers entstehen. Das Auswertungsmodul 231 der Recheneinheit 23 weist diesem Abschnitt entsprechend eine Gehbewegung zu und entfernt die zugehörigen Daten aus den Bewegungsdaten. Analog dazu treten ab etwa 28 Sekunden regelmässige Peaks b) auf. Auch dieser Abschnitt wird vom Auswertungsmodul 231 der Recheneinheit 23 als Sekundärbewegungen ausgefiltert. Somit verbleiben nach dem Filtern die Bewegungsdaten des Abschnitts zwischen 14 und 28 Sekunden als primäre Armbewegungen.

Während oder nach dem Ausfiltern berechnet die Recheneinheit 23 fortlaufend Magnituden c) der Bewegungsdaten. Dabei entsprechen jeweils pro Zeitpunkt die Magnituden der Wurzel aus der Summe der Quadrate von Werten in drei Richtungen eines dreidimensionalen Koordinatensystems. Die Magnituden werden von der Recheneinheit 21 über ein regelmässiges Zeitfenster von 20 Sekunden zu einem Aktivitätszähler (Activity Count) aufsummiert.

In der Vorrichtung 1 ist weiter ein Tagesbewegungsziel definiert und im Datenspeicher 24 abgelegt. Diese Definition erfolgt über das Smartphone 4, wenn die Funktionseinheit 2 und das Smartphone 4 verbunden sind. Auf dem Display 22 wird laufend ein Grad der Erreichung des Bewegungsziels dargestellt. In der Fig. 1 und der Fig. 2 hat der Benutzer 43% des Tagesbewegungsziels erreicht. Das Bewegungsziel kann von der Recheneinheit 23 aufgrund von gespeicherten Bewegungsdaten automatisch nach oben angepasst werden.

Zudem wertet die Recheneinheit die gefilterten Bewegungsdaten aus und warnt den Benutzer, wenn die ausgewerteten Bewegungsdaten über einen vordefinierten Ruhezeitraum einen vordefinierten Grenzwert nicht überschreiten. Der Benutzer kann dazu die Funktionseinheit 2 so einstellen, dass eine visuelle Information auf dem Display 22, ein akustischer Alarm vom Lautsprecher 282 und/oder eine Vibration durch den Vibrator 281 als Warnung erzeugt wird.

Über die Verbindung mit dem Smartphone 4 mittels des Funkschnittstellenadapters 29 kann die Funktionseinheit 21 mittels des Smartphones 4 eingestellt werden. Zudem können die Bewegungsdaten und andere Informationen auf das Smartphone 4 übertragen werden. Das Smartphone 4 kann dann die Bewegungsdaten darstellen und weiter verarbeiten.

Obwohl die Erfindung mittels der Figuren und der zugehörigen Beschreibung dargestellt und detailliert beschrieben ist, sind diese Darstellung und diese detaillierte Beschreibung illustrativ und beispielhaft zu verstehen und nicht als die Erfindung einschränkend. Um die Erfindung nicht zu verklären, können in gewissen Fällen wohlbekannte Strukturen und Techniken nicht im Detail gezeigt und beschrieben sein. Es versteht sich, dass Fachleute Änderungen und Abwandlungen machen können, ohne den Umfang der folgenden Ansprüche zu verlassen. Insbesondere deckt die vorliegende Erfindung weitere Ausführungsbeispiele mit irgendwelchen Kombinationen von Merkmalen ab, die von den explizit beschriebenen Merkmalskombinationen abweichen können.

Die vorliegende Offenbarung umfasst auch Ausführungsformen mit jeglicher Kombination von Merkmalen, die vorstehend oder nachfolgend zu verschiedenen Ausführungsformen genannt oder gezeigt sind. Sie umfasst ebenfalls einzelne Merkmale in den Figuren, auch wenn sie dort im Zusammenhang mit anderen Merkmalen gezeigt sind und/oder vorstehend oder nachfolgend nicht genannt sind. Auch können die in den Figuren und der Beschreibung beschriebenen Alternativen von Ausführungsformen und einzelne Alternativen deren Merkmale vom Erfindungsgegenstand beziehungsweise von den offenbarten Gegenständen ausgeschlossen sein. Die Offenbarung umfasst Ausführungsformen, die ausschliesslich die in den Ansprüchen beziehungsweise in den Ausführungsbeispielen beschriebenen Merkmale umfasst sowie auch solche, die zusätzliche andere Merkmale umfassen.

Im Weiteren schliesst der Ausdruck "umfassen" und Ableitungen davon andere Elemente oder Schritte nicht aus. Ebenfalls schliesst der unbestimmte Artikel "ein" bzw. "eine" und Ableitungen davon eine Vielzahl nicht aus. Die Funktionen mehrerer in den Ansprüchen aufgeführter Merkmale können durch eine Einheit beziehungsweise einen Schritt erfüllt sein. Die Begriffe "im Wesentlichen", "etwa", "ungefähr" und dergleichen in Verbindung mit einer Eigenschaft beziehungsweise einem Wert definieren insbesondere auch genau die Eigenschaft beziehungsweise genau den Wert. Die Begriffe "etwa" und "ungefähr" im Zusammenhang mit einem gegebenen Zahlenwert oder -bereich kann sich auf einen Wert beziehungsweise Bereich beziehen, der innerhalb 20%, innerhalb 10%, innerhalb 5% oder innerhalb 2% des gegebenen Werts beziehungsweise Bereichs liegt.

Ein Computerprogramm kann auf einem geeigneten Medium gespeichert sein und/oder vertrieben werden, wie beispielsweise auf einem optischen Speichermedium oder einem Festmedium, das zusammen mit oder als Teil von anderer Hardware bereitgestellt wird. Es kann auch in anderer Form vertrieben werden, wie beispielsweise über das Internet oder andere verkabelte oder unverkabelte Telekommunikationssysteme. Insbesondere kann ein Computerprogramm beispielsweise ein auf einem computerlesbaren Medium gespeichertes Computerprogrammprodukt sein, das dazu ausgestaltet ist, ausgeführt zu werden, um ein Verfahren zu implementieren, insbesondere das erfindungsgemässe Verfahren. Alle Bezugszeichen in den Ansprüchen sind nicht als den Umfang der Ansprüche einschränkend zu verstehen.

## Patentansprüche

1. Vorrichtung (1) zur Erfassung und Auswertung von Bewegungen eines Benutzers, die eine Recheneinheit (23), einen Datenspeicher (24), eine Sensoreinheit (25) mit mindestens einem biometrischen Sensor und eine Befestigungseinrichtung (3) zur Befestigung der Sensoreinheit (25) an einem Arm des Benutzers umfasst, wobei die Recheneinheit (23) zur Erfassung von Bewegungsdaten aus durch die Sensoreinheit (25) erzeugten Bewegungssignalen und zur Speicherung der Bewegungsdaten im Datenspeicher (24) ausgestaltet ist, wobei
die Vorrichtung (1) ein Auswertungsmodul (231) umfasst, mit dem Sekundärbewegungsdaten, die passive Armbewegungen des Benutzers repräsentieren, aus den Bewegungsdaten filterbar sind, und
das Auswertungsmodul (231) dazu ausgestaltet ist, fortlaufend Magnituden der Bewegungsdaten zu berechnen,
wobei das Auswertungsmodul (231) dazu ausgestaltet ist, die aus den Bewegungsdaten berechneten Magnituden in einer Fensterfunktion zu bearbeiten,
wobei das Auswertungsmodul (231) dazu ausgestaltet ist, mittels Fourier-Transformation und insbesondere schneller Fourier-Transformation periodische Muster in den mit der Fensterfunktion bearbeiteten Magnituden zu identifizieren,
**dadurch gekennzeichnet, dass** das Auswertungsmodul (231) dazu ausgestaltet ist,
Schritte zu zählen, falls ein periodisches Muster identifiziert wird, und
Armaktivitäten zu zählen, falls kein periodisches Muster identifiziert wird.

2. Vorrichtung (1) nach Anspruch 1, die eine Warneinrichtung (28) umfasst, wobei die Recheneinheit (23) dazu ausgestaltet ist, die gefilterten Bewegungsdaten auszuwerten und die Warneinrichtung (28) zu aktivieren, wenn die ausgewerteten Bewegungsdaten über einen vordefinierten Ruhezeitraum einen vordefinierten Grenzwert nicht überschreiten.

3. Vorrichtung (1) nach Anspruch 2, bei der die Warneinrichtung (28) eine grafische Anzeige (22), einen Lautsprecher (282), eine Vibrationsstruktur (281), einen Muskelstimulator oder eine Kombination davon umfasst.

4. Vorrichtung (1) nach Anspruch 2 oder 3, die eine Montageeinrichtung (3) zur Befestigung der Warneinrichtung (28) an einem Finger oder einem Handballen des Benutzers umfasst.

5. Vorrichtung (1) nach einem der vorangehenden Ansprüche, bei der das Auswertungsmodul (231) einen Hochpassfilter und/oder einen Tiefpassfilter umfasst, mit dem beziehungsweise mit denen die Bewegungsdaten vorfilterbar sind.

6. Vorrichtung (1) nach Anspruch 5, bei der der Hochpassfilter beziehungsweise der Tiefpassfilter des Auswertungsmoduls auf einen Wert zwischen 0.1 Hz und 1 Hz und insbesondere auf einen Wert von etwa 0.3 Hz eingestellt ist.

7. Vorrichtung (1) nach einem der vorangehenden Ansprüche, bei der die Bewegungsdaten pro Zeitpunkt jeweils Werte von Bewegungen in drei Richtungen eines dreidimensionalen Koordinatensystems umfassen, und bei der die Magnituden jeweils der Wurzel aus der Summe der Quadrate der Werte in den drei Richtungen des gleichen Zeitpunkts entsprechen.

8. Vorrichtung (1) nach einem der vorangehenden Ansprüche, bei der das Auswertungsmodul (231) dazu ausgestaltet ist, einen Aktivitätszähler zu berechnen, der der Summe aller Magnituden oder Integrale in einem vordefinierten Zeitfenster entspricht, wobei das vordefinierte Zeitfenster vorzugsweise in einem Bereich von zwischen 0.5 Sekunden bis zehn Minuten und insbesondere in einem Bereich von zwischen zwei Sekunden bis fünf Minuten liegt.

9. Vorrichtung (1) nach einem der vorangehenden Ansprüche, bei dem der mindestens eine biometrische Sensor der Sensoreinheit (25) einen Beschleunigungssensor (253), ein Gyroskop (252), einen Magnetometer (254), einen Drucksensor, einen GPS-Sensor (251), einen Muskelaktivitätssensor oder eine Kombination davon umfasst.

10. Vorrichtung (1) nach einem der vorangehenden Ansprüche, bei der die Sensoreinheit (25) zum Abtasten in einer Frequenz von mindestens 10 Hz oder von mindestens 20 Hz und insbesondere von in einem Bereich zwischen etwa 50 Hz bis etwa 100 Hz eingestellt ist.

11. Verfahren zur Erfassung und Auswertung von Bewegungen eines Benutzers mittels einer Vorrichtung nach einem der vorangehenden Ansprüche, umfassend
Befestigen der Sensoreinheit (25) an einem Arm des Benutzers,
Erfassen von Bewegungsdaten durch die Recheneinheit (23) aus durch die Sensoreinheit (25) erzeugten Bewegungssignalen, und
Speichern der Bewegungsdaten durch die Recheneinheit (23) in dem Datenspeicher (24),
Ausfiltern von Sekundärbewegungsdaten, die passive Armbewegungen des Benutzers repräsentieren, durch das Auswertungsmodul (231) aus den Bewegungsdaten, indem fortlaufend Magnituden der Bewegungsdaten berechnet werden,
wobei die aus den Bewegungsdaten berechneten Magnet tuten durch das Auswertungsmodul (231) in einer Fensterfunktion bearbeitet werden, wobei mittels Fourier-Transformation und insbesondere schneller Fourier-Transformation durch das Auswertungsmodul (231) periodische Muster in mit der Fensterfunktion bearbeiteten Magnituden identifiziert werden,
**dadurch gekennzeichnet, dass** durch das Auswertungsmodul (231)
Schritte gezählt werden, falls ein periodisches Muster identifiziert wird, und
Armaktivitäten gezählt werden, falls kein periodisches Muster identifiziert wird.

12. Verfahren nach Anspruch 11, umfassend
Befestigen einer weiteren Sensoreinheit (25) an einem zweiten Arm des Benutzers, wobei die weitere Sensoreinheit (25) mindestens einen biometrischen Sensor aufweist,
Erfassen von weiteren Bewegungsdaten aus durch die weitere Sensoreinheit (25) erzeugten Bewegungssignalen,
Speichern der weiteren Bewegungsdaten in einem Datenspeicher (24),
Ausfiltern von Sekundärbewegungsdaten aus den weiteren Bewegungsdaten, und
Vergleichen der ausgefilterten Bewegungsdaten mit den ausgefilterten weiteren Bewegungsdaten.

13. Verfahren nach Anspruch 11 oder 12, das ein Definieren eines Bewegungsziels, insbesondere eines Tages- oder Wochenbewegungsziels und ein Darstellen eines Grads der Erreichung des Bewegungsziels umfasst.

14. Verfahren nach Anspruch 13, bei dem das Bewegungsziel aufgrund von gespeicherten Bewegungsdaten automatisch definiert und insbesondere automatisch nach oben oder nach unten angepasst wird.

## Claims

1. A device (1) for acquiring and evaluating movements of a user, which comprises a computing unit (23), a data memory (24), a sensor unit (25) with at least one biometric sensor, and a fixing device (3) for fixing the sensor unit (25) to an arm of the user, wherein the computing unit (23) is designed for acquiring movement data from movement signals generated by the sensor unit (25) and for storing the movement data in the data memory (24), wherein
the device (1) comprises an evaluation module (231) by which secondary movement data, which represent passive arm movements of the user, can be filtered out of the movement data, and
the evaluation module (231) is designed to continuously calculate magnitudes of the movement data,
wherein the evaluation module (231) is designed to process the magnitudes calculated from the movement data in a window function,
wherein the evaluation module (231) is designed to identify periodic patterns in the magnitudes processed with the window function by means of Fourier transformation and in particular fast Fourier transformation,
**characterized in that** the evaluation module (231) is designed
to count steps, if a periodic pattern is identified, and
to count arm activities, if no periodic pattern is identified.

2. The device (1) according to claim 1, which comprises a warning device (28), wherein the computing unit (23) is designed to evaluate the filtered movement data and to activate the warning device (28) when the evaluated movement data do not exceed a predefined limit value over a predefined rest period.

3. The device (1) according to claim 2, in which the warning device (28) comprises a graphical display (22), a loudspeaker (282), a vibration structure (281), a muscle stimulator or a combination thereof.

4. The device (1) according to claim 2 or 3, which comprises a mounting device (3) for fixing the warning device (28) to a finger or a palm of the user.

5. The device (1) according to any of the preceding claims, in which the evaluation module (231) comprises a high-pass filter and/or a low-pass filter by one or both of which the movement data can be prefiltered.

6. The device (1) according to claim 5, in which the high-pass filter or the low-pass filter of the evaluation module is set on a value between 0.1 Hz and 1 Hz, and in particular on a value of about 0.3 Hz.

7. The device (1) according to any of the preceding claims, in which the movement data each comprise values of movements in three directions of a three-dimensional coordinate system per point in time, and in which the magnitudes each correspond to the square root of the sum of the squares of the values in the three directions of the same point in time.

8. The device (1) according to any of the preceding claims, in which the evaluation module (231) is designed to calculate an activity counter which corresponds to the sum of all magnitudes or integrals in a predefined time window, wherein the predefined time window preferably lies in a range of between 0.5 seconds to ten minutes, and in particular in a range of between two seconds to five minutes.

9. The device (1) according to any of the preceding claims, in which the at least one biometric sensor of the sensor unit (25) comprises an acceleration sensor (253), a gyroscope (252), a magnetometer (254), a pressure sensor, a GPS sensor (251), a muscle activity sensor or a combination thereof.

10. The device (1) according to any of the preceding claims, in which the sensor unit (25) is set for scanning in a frequency of at least 10 Hz or of at least 20 Hz, and in particular in a range between about 50 Hz to about 100 Hz.

11. A method for acquiring and evaluating movements of a user by means of a device according to any of the preceding claims, comprising
fixation of the sensor unit (25) to an arm of the user,
acquisition of movement data by the computing unit (23) from movement signals generated by the sensor unit (25), and
storage of the movement data by the computing unit (23) in the data memory (24),
filtering of secondary movement data, which represent passive arm movements of the user, by the evaluation module (231) out of the movement data by continuously calculating magnitudes of the movement data,
wherein the magnitudes calculated from the movement data are processed by the evaluation module (231) in a window function, wherein periodic patterns in magnitudes processed with the window function are identified by the evaluation module (231) by means of Fourier transformation and in particular fast Fourier transformation,
**characterized in that** steps are counted by the evaluation module (231) if a periodic pattern is identified, and arm activities are counted if no periodic pattern is identified.

12. The method according to claim 11, comprising
fixation of a further sensor unit (25) to a second arm of the user, wherein the further sensor unit (25) includes at least one biometric sensor,
acquisition of further movement data from movement signals generated by the further sensor unit (25),
storage of the further movement data in a data memory (24),
filtering of secondary movement data out of the further movement data, and
comparison of the movement data filtered out with the further movement data filtered out.

13. The method according to claim 11 or 12, which comprises a definition of a movement target, in particular a daily or weekly movement target, and a representation of a degree of reaching the movement target.

14. The method according to claim 13, in which the movement target is automatically defined due to stored movement data and in particular is automatically adapted upwards or downwards.

## Revendications

1. Dispositif (1) pour l'acquisition et l'évaluation de mouvements d'un usager, qui comprend une unité de calcul (23), une mémoire de données (24), une unité de capteur (25) avec au moins un capteur biométrique et un dispositif de fixation (3) pour la fixation de l'unité de capteur (25) sur un bras de l'usager, l'unité de calcul (23) étant conçue pour l'acquisition de données de mouvement à partir de signaux de mouvement générés par l'unité de capteur (25) et pour le stockage des données de mouvement dans la mémoire de données (24), dans lequel
le dispositif (1) comprend un module d'évaluation (231) avec lequel des données de mouvement secondaires, qui représentent des mouvements passifs du bras de l'usager, peuvent être filtrées à partir des données de mouvement, et
le module d'évaluation (231) est conçu pour calculer en continu des magnitudes des données de mouvement,
le module d'évaluation (231) étant conçu pour traiter les magnitudes calculées à partir des données de mouvement dans une fonction de fenêtre,
le module d'évaluation (231) étant conçu pour identifier, au moyen d'une transformation de Fourier et en particulier d'une transformation de Fourier rapide, des motifs périodiques dans les magnitudes traitées avec la fonction de fenêtre,
**caractérisé en ce que** le module d'évaluation (231) est conçu pour
compter les pas si un motif périodique est identifié, et
compter les activités des bras si aucun motif périodique n'est identifié.

2. Dispositif (1) selon la revendication 1, qui comprend un dispositif d'avertissement (28), l'unité de calcul (23) étant conçu pour évaluer les données de mouvement filtrées et pour activer le dispositif d'avertissement (28) lorsque les données de mouvement évaluées ne dépassent pas une valeur limite prédéfinie sur une période de repos prédéfinie.

3. Dispositif (1) selon la revendication 2, dans lequel le dispositif d'avertissement (28) comprend un affichage graphique (22), un haut-parleur (282), une structure vibrante (281), un stimulateur musculaire ou une combinaison de ceux-ci.

4. Dispositif (1) selon la revendication 2 ou 3, qui comprend un dispositif de montage (3) pour fixer le dispositif d'avertissement (28) sur un doigt ou une paume de l'usager.

5. Dispositif (1) selon l'une quelconque des revendications précédentes, dans lequel le module d'évaluation (231) comprend un filtre passe-haut et/ou un filtre passe-bas, avec lequel ou lesquels les données de mouvement peuvent être préfiltrées.

6. Dispositif (1) selon la revendication 5, dans lequel le filtre passe-haut ou le filtre passe-bas du module d'évaluation est réglé à une valeur comprise entre 0,1 Hz et 1 Hz et en particulier à une valeur d'environ 0,3 Hz.

7. Dispositif (1) selon l'une quelconque des revendications précédentes, dans lequel les données de mouvement comprennent, pour chaque instant, respectivement des valeurs de mouvements dans trois directions d'un système de coordonnées tridimensionnel, et dans lequel les magnitudes correspondent chacune à la racine de la somme des carrés des valeurs dans les trois directions du même instant.

8. Dispositif (1) selon l'une quelconque des revendications précédentes, dans lequel le module d'évaluation (231) est conçu pour calculer un compteur d'activité correspondant à la somme de toutes les magnitudes ou intégrales dans une fenêtre temporelle prédéfinie, la fenêtre temporelle prédéfinie étant de préférence comprise dans une plage comprise entre 0,5 seconde à dix minutes et en particulier dans une plage comprise entre deux secondes à cinq minutes.

9. Dispositif (1) selon l'une quelconque des revendications précédentes, dans lequel ledit au moins un capteur biométrique de l'unité de capteur (25) comprend un capteur d'accélération (253), un gyroscope (252), un magnétomètre (254), un capteur de pression, un capteur GPS (251), un capteur d'activité musculaire ou une combinaison de ceux-ci.

10. Dispositif (1) selon l'une quelconque des revendications précédentes, dans lequel l'unité de capteur (25) est réglée pour détecter à une fréquence d'au moins 10 Hz ou d'au moins 20 Hz et, en particulier, dans une plage comprise entre environ 50 Hz à environ 100 Hz.

11. Procédé d'acquisition et d'évaluation de mouvements d'un usager au moyen d'un dispositif selon l'une quelconque des revendications précédentes, comprenant
la fixation de l'unité de capteur (25) sur un bras de l'usager,
l'acquisition de données de mouvement par l'unité de calcul (23) à partir de signaux de mouvement générés par l'unité de capteur (25), et
le stockage des données de mouvement par l'unité de calcul (23) dans la mémoire de données (24),
le filtrage, par le module d'évaluation (231), de données de mouvement secondaires représentant des mouvements passifs du bras de l'usager à partir des données de mouvement en calculant en continu des magnitudes des données de mouvement,
dans lequel les magnitudes calculées à partir des données de mouvement sont traitées par le module d'évaluation (231) dans une fonction de fenêtre, des motifs périodiques dans des magnitudes traitées avec la fonction de fenêtre étant identifiés par le module d'évaluation (231) au moyen d'une transformation de Fourier et en particulier d'une transformation de Fourier rapide,
**caractérisé en ce que** des étapes sont comptées par le module d'évaluation (231) si un motif périodique est identifié, et des activités de bras sont comptées si aucun motif périodique n'est identifié.

12. Procédé selon la revendication 11, comprenant
la fixation d'une autre unité de capteur (25) sur un deuxième bras de l'usager, l'autre unité de capteur (25) comprenant au moins un capteur biométrique,
l'acquisition d'autres données de mouvement à partir de signaux de mouvement générés par l'autre unité de capteur (25),
le stockage des autres données de mouvement dans une mémoire de données (24),
le filtrage des données de mouvement secondaires à partir des autres données de mouvement, et
le comparaison des données de mouvement filtrées avec les autres données de mouvement filtrées.

13. Procédé selon la revendication 11 ou 12, qui comprend la définition d'un objectif de mouvement, en particulier un objectif de mouvement quotidien ou hebdomadaire, et la représentation d'un degré de réalisation de l'objectif de mouvement.

14. Procédé selon la revendication 13, dans lequel l'objectif de mouvement est défini automatiquement sur la base de données de mouvement stockées et en particulier est adapté automatiquement vers le haut ou vers le bas.
